# EUROPEAN PATENT APPLICATION

(11) **EP 2 689 788 A1**
(43) Date of publication of application: **29.01.2014**
(21) Application number: 13177046.3
(22) Date of filing: 18.07.2013
(51) Int. Cl.: A61L 2/10, A61L 2/20

(54) **Apparatus for sanitizing pillows or anything else contained in the apparatus**

(30) Priority: 27.07.2012 IT MI20121318
(71) Applicant: Alovisi, Guglielmo, 20060 Cassina De'Pecchi MI (IT)
(72) Inventor: Alovisi, Guglielmo, 20060 Cassina De'Pecchi MI (IT)
(74) Representative: Modiano, Micaela Nadia

(57) **Abstract**

An apparatus (1) for sanitizing pillows (12) and other material that can be contained therein, comprising a box-like body (2) adapted to contain at least one pillow (12) or other item to be sanitized, means (10, 11) for generating and releasing a sanitizing agent being provided in combination with forced hyper-ventilation means (8, 9) adapted to force the sanitizing agent to pass through the pillow (12) or other item and sanitize it.

## Description

The present invention relates to an apparatus for sanitizing pillows or other items. More particularly, the invention relates to an apparatus that makes it possible to sanitize superficially and in depth pillows and other material that can be contained therein, particularly suitable for hotels and environments in which pillows and other items are used by several people.

As is known, hotels, motels, residences, holiday villages, hospitals, nursing homes and the like, during linen changing, gather sheets, pillow cases, towels, etc., which are sent to the in-house laundry or often to external companies that wash them.

However, the pillows, which should be washed periodically, are certainly not washed between the stay of one guest in the hotel and the stay of the next guest.

Therefore, although it is normal to replace the pillowcase, the pillow itself can become the receptacle of fungi, bacteria, germs and mites if it is not subjected to periodic washing.

It is evident that the contact of the head of a person with the pillow or the contact of the mouth with said pillow can transmit to the pillow a large quantity of bacteria, which the pillow absorbs and which can then be retransmitted to the next person who uses the same pillow, albeit with a clean pillowcase.

The aim of the present invention is to provide an apparatus for sanitizing pillows or other items that makes it possible to sanitize with a simple operation the pillow and optionally also other material that might be contained therein, so as to offer to people a pillow that is always sanitized and deodorized without the need to replace it between one stay and the next.

Within this aim, an object of the present invention is to provide an apparatus for sanitizing pillows or other items that makes it possible to sterilize, sanitize and deodorize pillows or other items that can constitute a dangerous vehicle of contagion for respiratory health, skin health and allergy health of users.

Another object of the present invention is to provide an apparatus for sanitizing pillows or other items that is highly reliable, relatively simple to provide and has competitive costs.

This aim, as well as these and other objects that will become more apparent hereinafter, are achieved by an apparatus for sanitizing pillows or other items, **characterized in that** it comprises a box-like body adapted to contain pillows or other items to be sanitized, means for generating and releasing a sanitizing agent being provided in combination with forced hyper-ventilation means adapted to allow the sanitizing agent to pass through the pillow or other item and sanitize it.

Further characteristics and advantages of the invention will become more apparent from the description of preferred but not exclusive embodiments of the apparatus according to the present invention, illustrated by way of non-limiting example in the accompanying drawings, wherein:

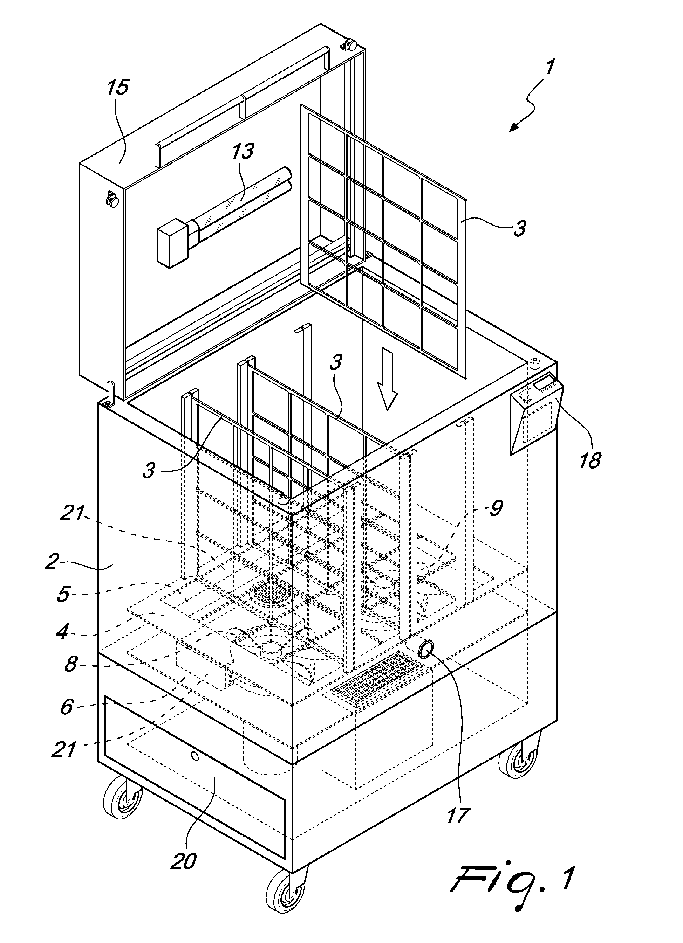

Figure 1 is a perspective view of the sanitizing apparatus according to the present invention;

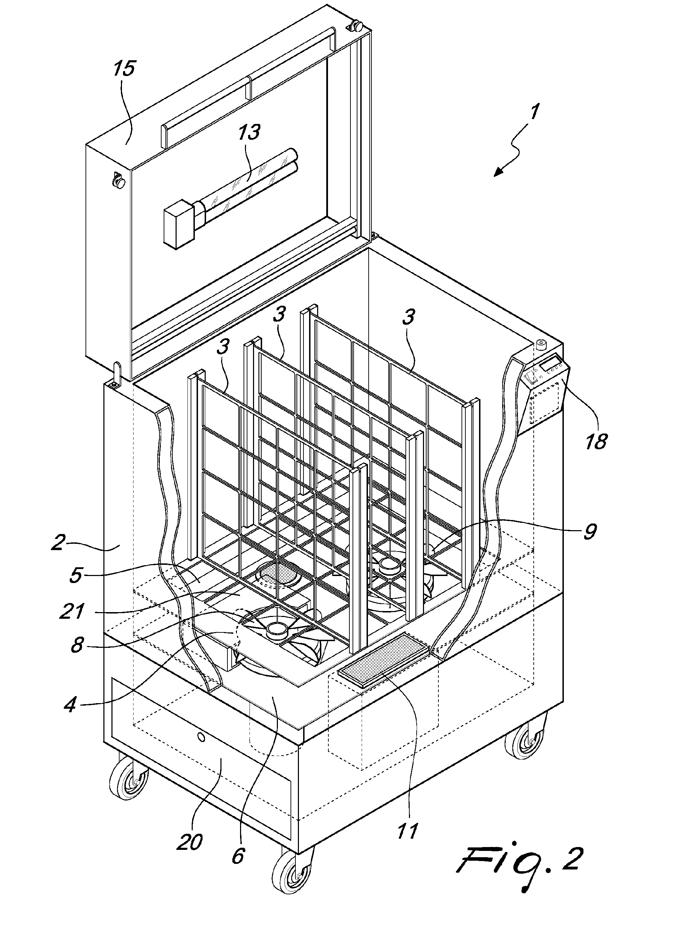

Figure 2 is a partially cutout view of the sanitizing apparatus according to the present invention;

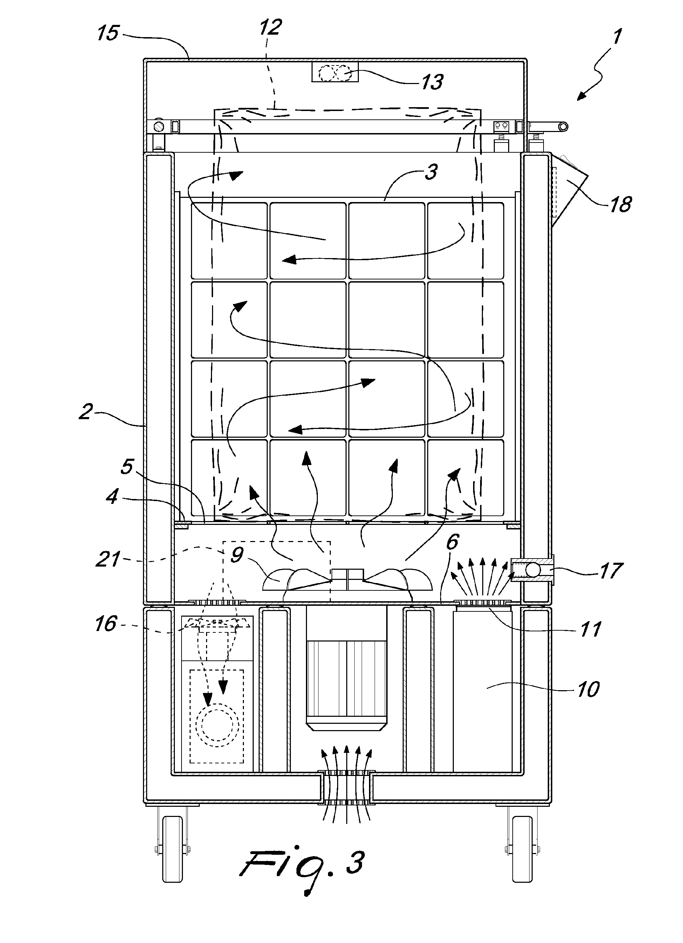

Figure 3 is a sectional side view of the apparatus according to the present invention;

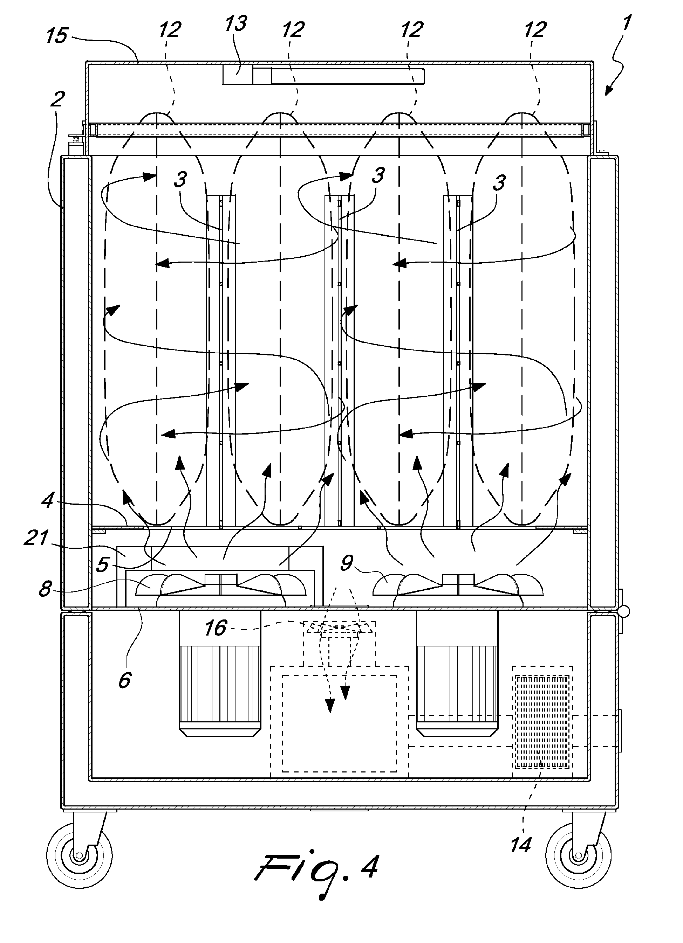

Figure 4 is a sectional front view of the apparatus according to the present invention;

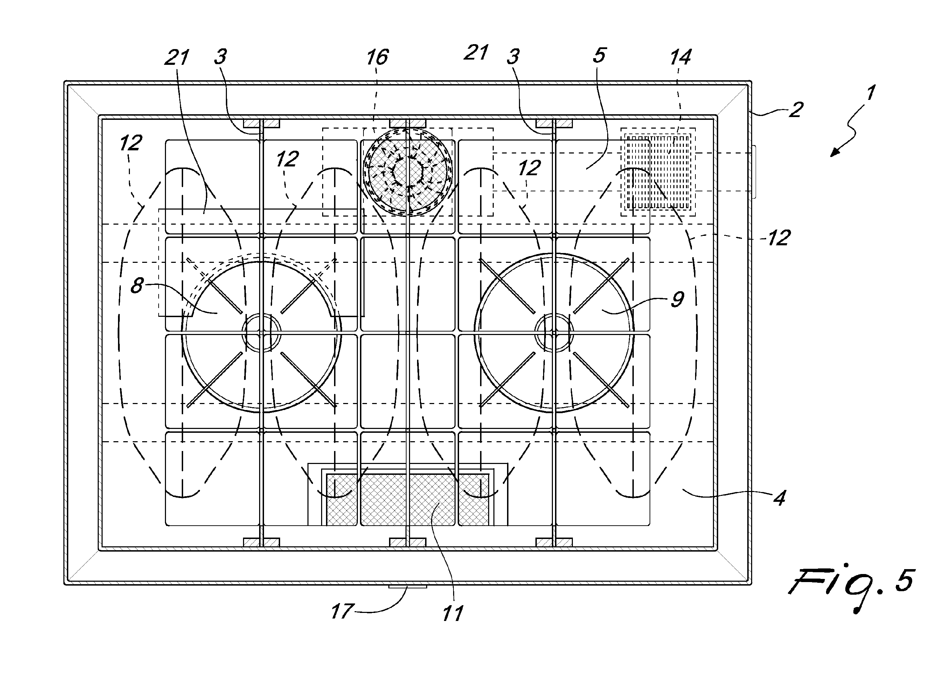

Figure 5 is a top plan view of the sanitizing apparatus according to the present invention; and
Figure 6 is a lateral elevation view of a variation of the apparatus according to the invention.

With reference to the figures, the sanitizing apparatus according to the invention, generally designated by the reference numeral 1, comprises a box-like body 2 that is adapted to contain at least one pillow 12 or other item to be sanitized. Conveniently, the box-like body 2 is divided by a plurality of partitions 3 adapted to form accommodation compartments for pillows or other items.

The bottom of the box-like body 2 is provided with a plate-like element 4, on which the partitions 3 rest, and is provided with a plurality of openings 5. A flat or inclined bottom 6 is provided under the plate-like element 4 (the inclined bottom is not shown in the figures).

Forced hyper-ventilation means, for example a pair of fans 8 and 9, driven by an electric motor for each fan, are provided above the bottom 6.

The apparatus according to the invention further comprises at least one source 10 of a sanitizing agent, such as for example a sanitizing gas such as ozone and the like, and means 11 for releasing said sanitizing gas toward the region of the box-like body 2 that is occupied by the pillows 12 or other items to be sanitized.

The apparatus may further comprise at least one lamp of the UV-C type or the like, designated by the reference numeral 13, for an additional treatment. The forced hyper-ventilation produced by the fans 8 and 9 allows the deep penetration of the sanitizing, sterilizing agent, such as for example ozone, through the pillows 3 to be sanitized.

At the bottom of the box-like body 2 there are filtering means 14 adapted to filter the ozone or sanitizing gas that is used and release it into the atmosphere, partially or totally converted into oxygen.

Upstream of the filtering means 14 there are suction means 16 adapted to convey toward the filtering means 14 the ozone-treated air contained in the box-like body 2.

Moreover, the use of ozone has a whitening effect on the pillows, an oxygenating effect for the environment, and a deodorizing and refreshing effect for the pillow.

The box-like body 2 is provided conveniently with a covering element 15, which allows complete closure of the box-like body during treatment with the sanitizing agent.

For allowing correct suction of the air contained within the box-like body 2, there is at least one check valve 17 that makes it possible to connect the outside environment to the inside of the box-like body 2.

Finally, the apparatus according to the invention is provided with a control keypad 18, associated with one or more electronic boards, for starting and stopping the apparatus and for setting operating parameters thereof.

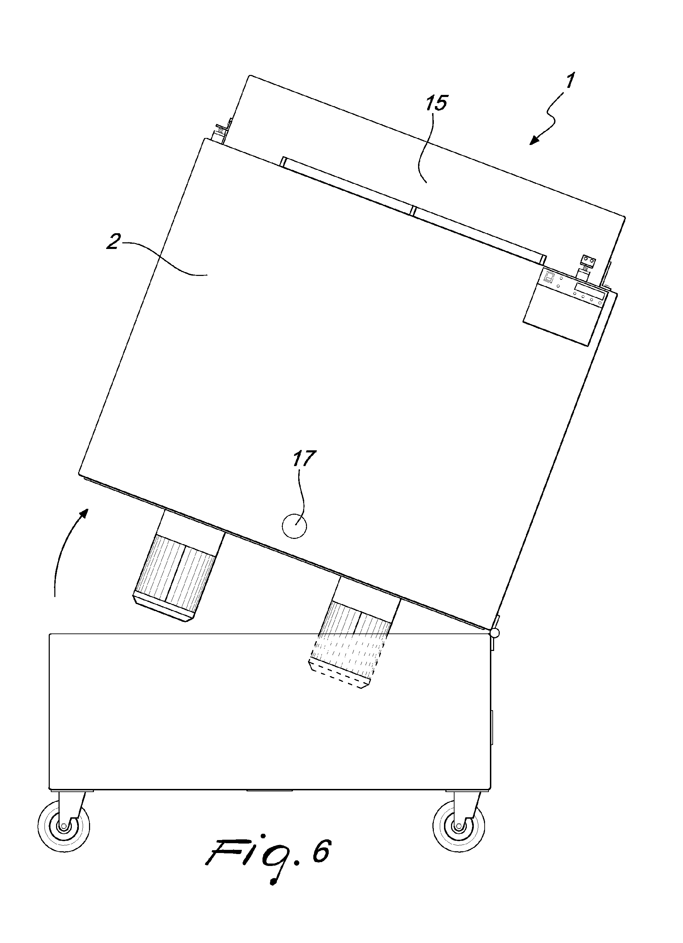

The apparatus according to the invention may optionally also be provided with a door 20, which allows access to the various components for maintenance and/or cleaning operations or a system for lifting and opening the body 2 from the base, as shown in Figure 6.

The base is connected to the body 2 with the interposition of gaskets that provide a hermetic seal of the base portion.

The apparatus of the invention may also comprise a humidifier 21 that is preferably accommodated near the bottom of the apparatus, around one of the fans 8,9.

Essentially, therefore, the apparatus according to the invention makes it possible to sanitize pillows or other items by means of the forced hyper-ventilation of a sanitizing agent such as ozone, which penetrates through the pillow or other item and sanitizes it.

The sanitizing effect is increased by the mechanical action that is created on the pillows by the friction of the air flow (generated by the fans) and the partitions 3 that define the compartments inside which the pillows are accommodated.

The hyper-ventilation allows to drastically reduce the humidity rate and also reduces the ozone rate. Due to the above, the sanitizing cycle of the apparatus can be very short.

The apparatus is made of a polyethylene based material: the use of polyethylene allows to attract on the inner walls of the apparatus the particulate that is extracted from the pillow by the sanitizing treatment. The inner walls of the apparatus can then be cleaned by a simple duster.

In this manner, hotels, hospitals, nursing homes, holiday villages and the like can clean safely the pillows or other items between the stay of one user and the next stay, without necessarily having to change the pillow, which by the way normally does not occur, and thus raising the health standards with respect to current ones.

The treatment that is performed with the apparatus according to the present invention is quick since, for example, with a cycle of about 5 minutes and 30 seconds the pillows are sanitized.

In practice it has been found that the sanitizing apparatus according to the present invention achieves fully the intended aim and objects.

The apparatus thus conceived is susceptible of numerous modifications and variations, all of which are within the scope of the appended claims. All the details may further be replaced with other technically equivalent elements.

In practice, the materials used, as well as the contingent shapes and dimensions, may be any according to the requirements and the state of the art.

The disclosures in Italian Patent Application No. MI2012A001318 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus (1) for sanitizing pillows or other items, **characterized in that** it comprises a box-like body (2) adapted to contain at least one pillow (12) or other item to be sanitized, means (10, 11) for generating and releasing a sanitizing agent being provided in combination with forced hyper-ventilation means (8, 9) adapted to allow the sanitizing agent to pass through the pillow (12) or other item and sanitize it.

2. The apparatus according to claim 1, **characterized in that** said forced hyper-ventilation means (8, 9) comprise at least one (8, 9) fan driven by an electric motor.

3. The apparatus according to claim 1, **characterized in that** said sanitizing agent is ozone.

4. The apparatus according to one or more of the preceding claims, **characterized in that** it comprises, at the bottom of said box-like body (2), a plate-like element (4) which is provided with openings (5) and below which the forced hyper-ventilation means (8, 9) are provided.

5. The apparatus according to one or more of the preceding claims, **characterized in that** it comprises, at the bottom of said box-like body (2), below the plate-like element (5) and the forced hyper-ventilation means (8, 9), a bottom (6) provided with openings for discharge toward filtering means (14).

6. The apparatus according to one or more of the preceding claims, **characterized in that** said discharge openings are connected to suction means (16), which in turn are connected to said filtering means (14) for filtering said sanitizing agent.

7. The apparatus according to one or more of the preceding claims, **characterized in that** it comprises a covering element (15) adapted to be pivoted to said box-like body (2) and to close completely said box-like body (2) when the sanitizing treatment is in operation.

8. The apparatus according to one or more of the preceding claims, **characterized in that** said box-like body (2) is divided into a plurality of compartments by means of partitions (3).

9. The apparatus according to one or more of the preceding claims, **characterized in that** it also comprises at least one lamp (13) of the UV-C type for the additional treatment of said pillows (12) or other items.

10. The apparatus according to one or more of the preceding claims, **characterized in that** said filtering means are connected to the environment that lies outside said box-like body.

11. The apparatus according to one or more of the preceding claims, **characterized in that** it comprises a humidifier (21).
